# EUROPEAN PATENT APPLICATION

(11) **EP 0 675 107 A1**
(43) Date of publication of application: **04.10.1995**
(21) Application number: 95104523.6
(22) Date of filing: 28.03.1995
(51) Int. Cl.: C07C 303/16, C07C 309/04, C07C 309/80

(54) **Pressurized production of alkanesulfonyl chloride and alkanesulfonic acid**

(30) Priority: 31.03.1994 US 221222
(71) Applicant: ELF ATOCHEM NORTH AMERICA, INC., Philadelphia Pennsylvania 19102-3222 (US)
(72) Inventor: Schon, Steven G., Strafford, Pennsylvania 19087 (US); Wheaton, Gregory A., Swedesboro, New Jersey 08085 (US); Overgaard, Thomas H., Redford, Michigan 48240 (US)
(74) Representative: Kraus, Walter, Dr.

(57) **Abstract**

A process for coproducing alkanesulfonyl chloride (ASC) or alkanesulfonic acid (ASA) and hydrochloric acid of reduced contamination with ASC, wherein a compound of the formula RSX, where X is hydrogen or -SR¹ and R and R¹ are alkyl radicals having from 1 to 20 carbon atoms, is reacted with chlorine in the presence of aqueous hydrochloride at an elevated pressure.

## Description

### BACKGROUND OF THE INVENTION

This invention is a process for the production of content along with the coproduction of alkanesulfonyl chloride (ASC) and/or alkanesulfonic acid (ASA), wherein a compound of the formula RSX, X being hydrogen or -SR¹ and R and R¹ are alkyl groups, is reacted with chlorine in an aqueous hydrochloric acid medium at an elevated pressure. More particularly, it relates to the process of reacting an alkyl mercaptan or alkyl disulfide with chlorine in an aqueous hydrogen chloride medium at a temperature of from about -10 to about 115°C and at a pressure of at least 1.05 atmospheres (absolute) up to 81.6 atm. abs. (the critical pressure of HCl), recovering an alkanesulfonyl chloride and/or alkanesulfonic acid, and, optionally, separately recovering an aqueous hydrochloric acid product having a substantially reduced alkanesulfonyl chloride content.

### The Prior Art

It is well known to manufacture either alkanesulfonyl chloride (ASC), alkanesulfonic acid (ASA) and mixtures thereof by reacting an alkyl mercaptan or an alkyl disulfide with chlorine in an aqueous hydrochloric acid medium at a reaction temperature ranging from about -10° up to about 115°C. In the known process, incondensible hydrogen chloride vapor is produced and passes upward with water vapor and some entrained ASA and/or ASC into a condenser where it is removed overhead. Entrained ASA is present as an aerosol while entrained ASC is a combination of ASC vapors (major contributor) and ASC aerosol (minor contributor). References disclosing these processes include, for example, U.S. Patent No. 3,626,004 issued December 7, 1971, U.S. Patent No. 3,600,136 issued August 17, 1971 and U.K. Patent No. 1,350,328 published April 18, 1974.

The prior art teaches that it is desirable to carry out the described processes at or below atmospheric pressure to facilitate overhead removal of gaseous products from the reactor. It is taught that residual hydrogen chloride, along with other volatile impurities, can be removed from the ASC and/or ASA products by vacuum or steam stripping.

The hydrogen chloride vapor co-produced overhead in accordance with the prior art methods contains significant quantities [thousands of parts per million (ppm] of ASC, due to the stripping effect of hydrogen chloride vapor emerging from the reactor. In end-uses for the hydrochloric acid coproduct, such as steel pickling, ASC is decomposed by the presence of ferrous ions, releasing objectionable odors. Consequently, it is necessary to further treat the hydrochloric acid coproduct to decrease its ASC levels to reduce or eliminate its odor-forming tendency.

### Statement of the Invention

This invention is a process for the continuous preparation of alkanesulfonyl chloride or alkanesulfonic acid comprising reacting a compound of the formula RSX, where X is hydrogen or the radical -SR¹ and R and R¹ are alkyl groups having from 1 to 20 carbon atoms, with chlorine in an aqueous hydrogen chloride medium at a temperature ranging from -10 to 115°C, and at a pressure of 1.05 atmospheres (atm.) absolute (abs.), or about 1.04 bars, up to 81.6 atm. abs., or about 80.54 bars.

### Detailed Description of the Invention

The process disclosed herein involves a pressurized reaction of a compound of the formula RSX where X is hydrogen or the radical -SR¹ and R and R¹ are alkyl groups having from 1 to 20 carbon atoms, preferably from 1 to 8 carbon atoms including, for example, methyl, ethyl, propyl, butyl, hexyl and octyl. The compounds represented by RSX are alkyl mercaptans and alkyl disulfides. The RSX compound is reacted with chlorine gas in an aqueous hydrogen chloride medium, preferably concentrated hydrochloric acid. While the reaction may be initiated in pure water or dilute hydrochloric acid, the aqueous medium will shortly become concentrated hydrochloride acid due to the evolution of gaseous hydrogen chloride in the aqueous medium from the reaction of the RSX compound and chlorine.

Some of the prior art requires the process to be carried out at reactant feed rates sufficient to produce vigorous turbulence while other teachings employ static or dynamic mixing means. While the process of this invention does not rely on such limitations to obtain improvement, such prior art practices may be beneficially employed. The reactants are preferably injected to the reactor in at least stoichiometic amounts, however, a slight excess of chlorine, e.g., 5%, may be desirable.

In a preferred embodiment, the feedrate of the RSX compound to the pressurized reaction ranges from 0.005 to 12 pound-mole per hour-foot squared (lbmole/hr.-ft²) of cross-sectional area of the "active" zone of the reactor, more preferably, from 0.5 to about 8.0 lbmole/ hr.-ft.².

This reaction, when carried out at a temperature within the range of -10 to 50°C, preferably 10 to 35°C, produces predominantly an ASC product with hydrogen chloride recovered overhead. At temperatures in the range of 85°C to 115°C, preferably 95 to 110°c, this reaction produces predominantly ASA coproduct with a small amount of ASC. A distinct mixture of ASC and ASA is obtained when the reaction temperature is between 50 and 85°C.

As previously stated, the known processes for producing ASC, ASA or mixtures thereof, coproduce hydrogen chloride (HCl) vapor, which is absorbed into water or water vapor to yield hydrochloric acid. This coproduct is recovered as a vapor overhead and contains undesirable quantities of ASC which the emerging HCl vapor strips from the reactor.

The process described above is unexpectedly improved by operation under pressure, and where the equipment is modified to permit the reaction to run under pressurized conditions. The pressure under which the process is run ranges from 1.05 atm. abs., or about 1.06 bars, up to 81.6 atm. abs. or about 82.7 bars (the critical pressure of HCl). Preferably, the reaction pressure will range from about 1.6 atm. abs. up to about 10 atm. abs., more preferably, from 1.1 to 5 atm. abs.

The process of this invention provides an unexpected improvement in gas-liquid contact and solubilization of the reactant gases, improving raw material utilization in the reactor. The process also has the unexpected effect of reducing the amount of liquid that can condense-out of the vapor emerging from the reactor (one would normally expect an increase in the amount of condensibles as pressure is increased) . This allows the reactor to use smaller less costly auxiliary systems such as condensers and pumps.

The process of this invention, operated at super atmospheric pressure, even with only a moderate elevation in pressure, significantly reduces the contamination of the HCl co-product by ASC, i.e. pressurized operation mitigates the stripping effect of the HCl vapor emerging from the reactor.

Pressurized operation also improves the volumetric efficiency of the reactor, by increasing the liquid fraction in the ebulliating zone (zone of reactor in which the reaction of RSX with chlorine produces turbulence), increasing the effective residence time, or conversely, increasing the allowable throughput at a given residence time. Pressurization also promotes disengagement of the vapor from the liquid, reducing the likelihood of entraining liquid with the gaseous co-product. This, in turn, increases the practical upper limit on the reactor throughput before hydrodynamic effects (e.g., foaming, entrainment) limit reactor capacity.

On formation of a liquid crude product (ASC, ASA or mixtures thereof) it is depressurized to atmospheric or sub-atmospheric pressure, preferably at or above the reaction temperature, to devolatilize the additional hydrogen chloride that goes into solution as a result of the pressurized operation. The depressurized crude liquid reaction product is treated further, as described in the known art, to remove residual impurities. The HCl vapor liberated by the depressurization step is removed overhead and may be processed further by conventional means, to recover and/or further purify the HCl.

The vapors and gases exiting the top of the reactor pass to a condenser. The condensed liquid leaving the condenser is returned to the reactor. The HCl and other non-condensible gases (e.g., inerts, excess chlorine, or other gaseous reaction products), if any, leave the reactor system via a pressure-regulating device, used to maintain the system operating pressure, located in the vent-gas line after the condenser. The non-condensible gases may be processed further by conventional means, to recover, separate and/or further purify them.

In a preferred embodiment of the invention, a fractionation column is interposed between the reactor and the condenser, as follows:

The vapors and gases exiting the top of the reactor are sent to the bottom vapor inlet of a (pressurized) rectification column (e.g., packed or trayed fractionator). In the rectification column, the vapors are contacted with cooled condensed liquid, returning from the condenser, effecting a separation reducing (by distillative action) the residual concentration of less volatile species (such as ASC) in the vapor stream.

The purified vapor stream exits the top of the rectification column and is passed to a condenser. The condensed liquid leaving the condenser is returned as reflux to the top liquid inlet of the rectification column. The condensed liquid leaving the bottom of the rectification column is recycled to the reactor. The HCl and other non-condensible gases (e.g., inerts, excess chlorine, or other gaseous reaction products) if any, leave the reactor and rectification system via a pressure-regulating device used to maintain the system operating pressure, located in the vent-gas line after the condenser. The non-condensible gases may be processed further by conventional means, to recover, separate and/or purify them.

The apparatus described below was used in the following Examples 1-4:
The apparatus comprised a vertical tubular 4" internal diameter reactor constructed of sections of glass, glass-lined steel, and fluoropolymer-lined steel flanged pipe fittings (spools, tees, and crosses), with an overall length of 14 feet. The reactor was connected to an adjacent vertical ("side-arm") heat exchanger by means of an overflow conduit about 12 feet up from the bottom of the reactor, and by a recycle conduit about 2 feet above the bottom of the reactor. The reactor was configured for continuous operation.

For the preparation of methanesulfonic acid (methanesulfonyl chloride may be similarly prepared), the system was charged with a mixture containing about 70 vol % methanesulfonic acid (MSA), 26% water, and 4% hydrogen chloride (HCl). The reactor was filled to about 1 foot below the overflow conduit. Chlorine gas (99.5% pure) was sparged into the reactor on automatic flow control via a feed port about 3 1/2 feet from the bottom of the reactor (a drilled fluropolymer feed tube extending across the cross-section of the reactor served as the sparger). Vaporized methyl mercaptan (99 % pure) was sparged into the reactor on automatic flow control via a feed port about 5 feet from the bottom of the reactor (a drilled fluoropolymer feed tube extending across the cross-section of the reactor served as the sparger).

The reaction of chlorine with the mercaptan was very vigorous, evolving gaseous HCl, which was vented (along with unreacted chlorine and mercaptan) from the top of the reactor to a condenser, with the condensate being returned to the reactor, and the non-condensibles sent to a caustic scrubber via a pressure control valve. An automatic pressure controller maintained the top of the reactor at the target pressure.

The gas evolution created bubbles which expanded the reaction liquid above the level of the overflow conduit, inducing a circulation through the side-arm exchanger, which was used to remove the exothermic heat of reaction. Coolant flow to the side-arm exchanger was adjusted by an automatic temperature controller, to maintain the temperature of the (cooled) liquid recirculating to the reactor at the target temperature. [The reactor was operated above 80°C, so that the primary reaction product was the sulfonic acid (MSA), with a minor percentage of unhydrolyzed methanesulfonyl chloride (MSC)] which may be hydrolyzed to MSA by heating in the presence of water, e.g. in the steam stripping operation for MSA.

A portion of the recirculating reaction liquid was taken-off on automatic flow control via the bottom of the reactor as crude MSA product. The take-off rate was adjusted to maintain the target specific gravity. The liquid level in the reactor was maintained at about 2 inches above the overflow conduit, by feeding make-up water to the reactor on automatic level control. The makeup water was combined with the recycle condensate and introduced to the reactor via a liquid feed port about 2 1/2 feet above the bottom of the reactor.

The "active" zone of the reactor was the 7 foot long section of the reactor between the mercaptan feed port and the overflow conduit to the heat exchanger. The bottom 5 feet of the "active" zone was constructed of glass piping, to allow visual observation of the bubble dynamics in the active zone.

### Example 1a

In this example, the reaction temperature was 113 °C. The reactor top pressure was 1.5 psig (1.10 atm. abs.). The mercaptan feedrate was 5.6 lb/hr, and the chlorine feedrate was 24.8 lb/hr, corresponding to 0.44% excess chlorine. The crude take-off rate was adjusted to maintain the specific gravity of the reaction liquid at 1.38, corresponding to about 76 wt. % MSA in the crude product.

Analyses of the caustic scrubber liquid for the sodium salt of MSA (the reaction product of MSC with sodium hydroxide) revealed that a "yield loss" of 3.0 % of the sulfur value in the mercaptan feed, in the form of MSC, was carried-over with the HCl vapor taken from the reactor.

### Example 1b

In this example, the operating conditions were identical to Example 1a, except that the reaction was carried-out at a pressure of 11 psig (1.75 atm. abs.).

Analyses of the caustic scrubber liquid revealed that a "yield loss" of 0.7% of the sulfur value in the mercaptan feed, in the form of MSC, was carried-over with the HCl vapor taken overhead from the reactor.

Increasing the reactor pressure to 11 psig resulted in over a 4-fold reduction in MSC contamination of the HCl (compared to Example 1a).

### Example 2a

In this example, the reaction temperature was 113°C. The reactor top pressure was 1.5 psig (1.10 atm. abs.). The mercaptan feedrate was 5.6 lb/hr., and the chlorine feedrate was 25.5 lb/hr., corresponding to 3.28% excess chlorine. The crude take-off rate was adjusted to maintain the specific gravity of the reaction liquid at 1.38, corresponding to about 76 wt. % MSA in the crude product.

Analyses of the caustic scrubber liquid revealed that a "yield loss" of 4.2% of the sulfur value in the mercaptan feed, in the form of MSC, was carried-over with the HCl vapor taken overhead from the reactor.

### Example 2b

In this example, the operating conditions were identical to Example 2a, except that the reaction was carried-out at a pressure of 11 psig (1.75 atm. abs.).

Analyses of the caustic scrubber liquid revealed that a "yield loss" of 2.8% of the sulfur value in the mercaptan feed, in the form of MSC, was carried-over with the HCl vapor taken from the reactor.

Increasing the reactor pressure to 11 psig resulted in a 1/3 reduction in MSC contamination of the HCl (compared to Example 2a).

### Example 3a

In this example, the reaction temperature was 113°C. The reactor top pressure was 20.5 psig (2.40 atm. abs.). The mercaptan feedrate was 5.6 lb/hr. and the chlorine feedrate was 25.5 lb/hr, corresponding to 3.28% excess chlorine. The crude take-off rate was adjusted to maintain the specific gravity of the reaction liquid at 1.30, corresponding to about 58 wt. % MSA in the crude product.

Analyses of the caustic scrubber liquid revealed that a "yield loss" of 0.8% of the sulfur value in the mercaptan feed, in the form of MSC, was carried-over with the HCl vapor taken from the reactor.

The low carry-over of MSC with the HCl vapor in this example of pressurized operation at 20.5 psig (2.40 atm. abs.) is comparable to that seen with pressurized operation at 11 psig, as described in Example 1b.

### Example 3b

In this example, it was attempted to duplicate the operating conditions of Example 3a, except that the reaction was carried-out at a pressure of 0.1 psig (1.00 atm. abs.).

It was impossible to reach the target temperature of 113°C, since the reaction liquid began to boil at about 105°C. The boiling disrupted the normal reactor recirculation, rendering the operating uncontrollable.

These results demonstrate that pressurized operation extends the feasible range of operating conditions for the ASC/ASA reactions.

### Example 4

The flowrates of the condensed vapors being returned to the reactor in the examples above were measured with a rotameter. At the operating conditions of Example 1a (1.5 psig), the average condensate flow was about 20 lb/hr. At the operating conditions of Example 1b (11 psig), the average condensate flow was about 10-15 lb/hr.

This example shows that the amount of condensate produced in the reactor condenser decreases (rather than increases, as would be expected) with increasing operating pressure.

It has been the experience in a commercial plant producing methanesulfonyl chloride and methanesulfonic acid by the method described in the prior art mentioned herein, operating at near atmospheric pressure, that the volume of condensate produced limits the system capacity (condensate flow is greater than can be handled by the condenser and its piping). Hence, it is of practical importance that the volume of condensate can be reduced by operating the reactor and condenser system at elevated pressure, allowing increased production capacity.

The following example is set forth to demonstrate the use of a fractionating or rectifying column in the manufacture of ASC and ASA. The interposition of a fractionator between the reactor and condenser unexpectedly greatly decreases the amount of ASC entrained in the hydrogen chloride byproduct gases. This fractionating system will function advantageously with the superatmospheric pressure reaction of this invention as well as with vacuum and normal atmospheric pressure reactions of the prior art for the continuous preparation of alkanesulfonyl chloride and alkanesulfonic acid by the reaction of an alkyl mercaptan or alkyl disulfide with chlorine under the temperature conditions described herein. The following experiments were conducted at atmospheric pressure in the reactor.

### Example 5

The object of the following experiments was to determine the effectiveness of the several systems to reduce and recover entrained MSC.

A reactor was used consisting of a 500 ml., three-neck flask filled with 200 ml. of 36% aqueous hydrochloric acid (HCl) and 200 ml. of methanesulfonyl chloride (MSC). Mounted vertically atop the reactor flask and attached to one of the three necks was one of three types of tubular columns described hereinafter. The second neck was connected to a sintered glass frit of course porosity which sparged anhydrous HCl through a rotameter mounted atop an HCl supply cylinder into the reactor to simulate the HCl evolution which occurs during the reaction of methyl mercaptan with gaseous chlorine, in a concentrated aqueous HCl media. The HCl feed rate to the reactor was 1.57 l./min. (0.07 moles/min. or 2.5 grams/min.). The third neck of the flask was used to supply the aqueous HCl and MSC to the reaction zone.

In the first experiment (A), the tubular column attached vertically to the top of the reactor was an open column 60 cm. in length by 2.5 cm. in diameter. In the second experiment (B), the tubular column was of the same size as in experiment A but was packed with 0.5 cm ceramic saddles (functions as a crude fractionator). In the third experiment (C), the column was a 30 tray Oldershaw column.

The top of each column in experiments A, B and C was connected by tubing for gas transfer to the top of an adjacent "HCl absorber" consisting of a verticle glass tube of 52.5 cm. in length by 2.54 cm. in diameter packed with 6 mm. glass helices. Tube connections were positioned at the top and bottom of the HCl absorber to circulate water in the absorber. Distilled water was injected at the top of the absorber column, as was the gases emanating from the top of the intermediate tubular column. In each experiment, the water was recirculated through the HCl absorber until the specific gravity indicated a concentration of 32% HCl in water. The water was circulated for 56 minutes absorbing 141g. of HCl. The final HCl solution weighed 441 g. (141 g. HCl plus 300g. H₂0) and the final volume was 380 ml. The temperature of the MSC/aqueous HCl mixture was 25°C for each experiment.

When anhydrous HCl is passed through the MSC/aqueous HCl mixture, the HCl gas vaporizes/entrains MSC, as well as HCl/water mixture, at a rate dependent on temperature.

In experiment A, all entrained material entered the top of the HCl absorber from the open tubular column. 2.09g of MSC was dissolved in 441 g. of 32% HCl. The MSC concentration was 4751 parts per million (ppm) in the 32% concentrated aqueous HCl. The 2.09 g. of MSC was liberated by 141 of HCl gas which indicates an MSC concentration of 0.0148 g. of MSC per g. of HCl gas.
In experiment B, the same conditions were established as in experiment A but the open tubular column was packed with 0.5 cm. ceramic saddles and reflux was established. The reflux was slight, ≈ 0.5 ml/min. This was induced by the evaporation and subsequent condensation of the HCl/H₂O on the ceramic saddles. Under these conditions, 1.307 g. of MSC was liberated in 441 g. of 32% conc. aqueous HCl. The MSC concentration was 2971 ppm in the 32% HCl. The 1.307 g. of MSC was liberated by 141 g. of HCl gas which indicates an MSC concentration of 0.00927 g. of MSC/g. of HCl gas. This represents a reduction in the amount of MSC entrained in the HCl of 37.5% when compared to the experiment A results.

In experiment C, the same conditions were established as in experiment A but the open tubular column was replaced with the 30 tray, 2.54 cm. diameter Oldershaw column. This type of column provides enhanced gas-liquid contact. The tray design retains a small amount of liquid on each tray through which the gas must pass. The reflux was slight, ≈ 0.5 ml./min. This was induced by the evaporation and subsequent condensation of the HCl/H₂O on the trays. Under these conditions, 0.373 g. of MSC was dissolved in 440 g. of 32% concentrated aqueous HCl. The MSC concentration was 847 ppm in the 32% HCl. The 0.373 g. of MSC was liberated by 141 g. of HCl gas which indicates an MSC concentration of 0.0026 g. MSC/g. of HCl gas. This represents a reduction in the amount of MSC entrained in the HCl of 82.4% when compared to the experiment A results.

These experiments indicate the unexpectedly improved results in reduction of MSC entrained in the byproduct HCl gas when interposing a rectifier (fractionation) column between the reactor and condenser in the process of manufacturing alkanesulfonyl chloride (or alkanesulfonic acid) from an alkyl mercaptan or alkyl disulfide and chlorine in an aqueous medium.

While the process of this invention, as demonstrated in the foregoing examples, has been carried out in the apparatus as previously described, additional improvements in the process will be attained by the use of stationary (rigid, motionless) mixing elements or units packed within the "active" reaction zone of the reactor column. This type of mixing device is described in Bulletin KSM-6, Copyright 1991, Koch Engineering Company, Inc. and has been demonstrated to promote plug-flow in the continuous reaction process disclosed herein. The continuous process for the manufacture of alkanesulfonyl chloride and alkanesulfonic acid utilizing stationary mixing means is disclosed and claimed in U.S. patent application serial number 08/221,224 filed of even date herewith.

## Claims

1. A continuous process for the preparation of an alkanesulfonyl chloride or alkanesulfonic acid comprising reacting a compound of the formula RSX, where X is hydrogen or the radical -SR¹ and R and R¹ are alkyl groups having from 1 to 20 carbon atoms, with chlorine in an aqueous hydrogen chloride medium at a temperature ranging from -10 to 115°C, at a pressure ranging from 1.05 to 81.6 atmospheres absolute, and recovering alkanesulfonyl chloride, alkanesulfonic acid or mixtures thereof.

2. The process of claim 1 wherein hydrogen chloride vapor is recovered overhead.

3. The process of claim 2 wherein condensible vapors of the reaction are refluxed back to the reactor.

4. The process of claim 3 wherein other noncondensible gases of the reaction are recovered overhead with said hydrogen chloride vapor.

5. The process of claim 4 wherein reaction gases emitted overhead are rectified by passage in contact with condensed liquid of refluxed vapor before said liquid is returned to the reactor.

6. A continuous process for the preparation of an alkanesulfonyl chloride or alkanesulfonic acid comprising reacting a compound of the formula RSX, where X is hydrogen or the radical -SR¹ and R and R¹ are alkyl groups having from 1 to 8 carbon atoms, with chlorine in aqueous hydrochloric acid at a mole ratio of reactants up to about 5% excess chlorine and a feedrate of RSX compound ranging from 0.005 to 12 lbmole/hr - ft₂ of cross-section of reactor area, at a temperature ranging from -10 to 115°C, at a pressure ranging from 1.05 to 81.6 atm. abs., and recovering alkanesulfonyl chloride, alkanesulfonic acid or mixtures thereof.

7. The process of claim 6 wherein the RSX compound is methyl mercaptan.

8. The process of claim 6 wherein the RSX compound is dimethyl disulfide.

9. The process of claim 6 wherein hydrogen chloride vapor is recovered overhead.

10. The process of claim 6 wherein the feedrate of RSX compound ranges from 0.5 to about 8.0 lbmole/hr-ft², the mole ratio of reactants ranges from stoichiometric up to 5% excess chlorine, the temperature ranges from 10° to 115°C, and the pressure ranges from 1.6 to 10 atm. abs.

11. The process of claim 10 wherein hydrogen chloride vapor is recovered overhead.

12. The process of claim 11 wherein RSX is methyl mercaptan.

13. The process of claim 11 wherein RSX is dimethyl disulfide.

14. The process of claim 11 wherein condensible vapors from the reaction are refluxed back to the reactor.

15. The process of claim 14 wherein other noncondensible gases of the reaction are recovered overhead with the hydrogen chloride vapor.

16. The process of claim 15 wherein reaction gases emitted overhead are rectified by passage in contact with condensed liquid product of the refluxed vapors before said condensed liquid is returned to the reactor.

17. The process of claim 16 wherein RSX is methyl mercaptan.

18. The process of claim 17 wherein the reaction temperature ranges from 10° to 35°C.

19. The process of claim 17 wherein the reaction temperature ranges from 85° to 115°C.

20. The process of claim 16 wherein RSX is dimethyl disulfide.

21. A continuous process for the preparation of an alkanesulfonyl chloride or alkanesulfonic acid comprising reacting a compound of the formula RSX, where X is hydrogen or the radical -SR¹ and R and R¹ are alkyl groups having from 1 to 20 carbon atoms, with chlorine in an aqueous hydrogen chloride medium at a reaction temperature ranging from -10 to 115°C, rectifying reaction gases emitted overhead from the reactor, condensing the vapors of the reaction after rectification, and recovering alkanesulfonyl chloride, alkanesulfonic acid or mixtures thereof.

22. The process of claim 21 wherein said alkyl groups have from 1 to 8 carbon atoms.

23. The process of claim 22 wherein the reaction temperature ranges from 10 to 35°C.

24. The process of claim 22 wherein the reaction temperature ranges from 85 to 115°C.

25. The process of claim 21 wherein the reaction pressure ranges from 1.05 to 81.6 atmospheres absolute.

26. The process of claim 21 wherein the reaction pressure is about atmospheric.
